(19) Europäisches Patentamt
European Patent Office
Office européen des brevets

(11) EP 0 678 292 B1

(12) **FASCICULE DE BREVET EUROPEEN**

(45) Date de publication et mention de la délivrance du brevet:
**27.05.1998 Bulletin 1998/22**

(51) Int. Cl.$^6$: **A61K 7/42**

(21) Numéro de dépôt: **95400547.6**

(22) Date de dépôt: **14.03.1995**

(54) **Compositions cosmétiques filtrantes contenant un agent hydrophile acide et utilisation**

Filtrierende kosmetische Zusammensetzung enthaltend ein Hydrophilierungsmittel und Verwendung

Cosmetic filtering compositions containing a hydrophilic agent and uses thereof

(84) Etats contractants désignés:
**AT BE CH DE ES FR GB IT LI NL SE**

(30) Priorité: **19.04.1994 FR 9404634**

(43) Date de publication de la demande:
**25.10.1995 Bulletin 1995/43**

(73) Titulaire: **L'OREAL**
**75008 Paris (FR)**

(72) Inventeur: **Eteve, Martine**
**F-75013 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 390 682**
**GB-A- 2 185 019**
**GB-A- 2 225 013**

- **PATENT ABSTRACTS OF JAPAN vol. 12, no. 33 (C-472) (2880) 30 Janvier 1988 & JP-A-62 181 213 (KAO CORP.) 8 Août 1987**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 678 292 B1

Printed by Xerox (UK) Business Services
2.16.3/3.4

## Description

La présente invention concerne de nouvelles compositions cosmétiques filtrantes destinées à protéger la peau et les cheveux du rayonnement ultraviolet (compositions ci-après dénommées, plus simplement, compositions antisolaires) et présentant une bonne résistance à l'eau. Plus précisément encore, elle concerne des compositions cosmétiques résistantes à l'eau qui se présentent sous la forme d'émulsions de type huile-dans-eau et qui contiennent, à titre d'agents photoprotecteurs, des filtres UV hydrophiles comportant au moins un radical acide en association avec des charges inertes adsorbantes. L'invention concerne également l'utilisation desdites compositions pour la protection de la peau et/ou des cheveux contre le rayonnement ultraviolet.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Pour diverses raisons liées en particulier à un meilleur confort d'utilisation (douceur, émollience, facilité d'application et autres), les compositions antisolaires actuelles se présentent le plus souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques capables d'absorber selectivement les rayonnements UV nocifs, ces filtres étant sélectionnés en fonction de l'indice de protection recherché. Toutefois, l'un des problèmes rencontrés avec ce type de compositions antisolaires réside notamment dans le fait que, une fois appliquées sur la peau sous la forme d'un film par leurs utilisateurs, elles présentent une résistance à l'eau relativement faible.

Aussi, il a déja été proposé d'améliorer la résistance à l'eau des compositions filtrantes, en les formulant soit avec des polymères, soit encore dans un support de type émulsion eau-dans-huile en présence d'émulsionnants possédant un HLB (hydrophilie-lipophilie-balance) variant de 1 à 7, tel que cela est décrit respectivement dans les brevets US 5 041 281 et 5 047 232.

Les deux techniques mentionnées ci-dessus permettent effectivement d'améliorer la résistance à l'eau des compositions lorsque celles-ci renferment des filtres lipophiles. Il n'en est cependant pas de même avec les compositions renfermant des filtres à caractère hydrophiles, acides notamment, car ceux-ci disparaissent très facilement à l'eau, par baignade en mer ou en piscine, sous la douche ou lors de la pratique de sports nautiques ; ainsi les compositions solaires qui les contiennent, seuls ou associés aux filtres lipophiles, n'apportent plus la protection initiale recherchée dès lors que le substrat (peau ou cheveu) sur lequel elles ont été appliquées vient à être mis en contact avec l'eau. Il est alors nécessaire de procéder à une nouvelle application de produit antisolaire afin de conserver une protection convenable.

Le phénomène ci-dessus est particulièrement critique et pénalisant dans le cas où les compositions antisolaires sont appelées à se présenter sous la forme d'émulsions de type huile-dans-eau. Pour les émulsions huile-dans-eau ou pour les émulsions eau-dans-huile, les filtres hydrophiles sont présents dans la phase aqueuse, et les filtres lipophiles, dans la phase grasse. Comme indiqué précédemment, les émulsions huile-dans-eau sont bien plus appréciées par le consommateur que les émulsions eau-dans-huile, en raison notamment de leur toucher agréable (voisin de l'eau) et de leur présentation sous forme de lait ou de crème non gras ; cependant, et malheureusement, il s'avère également qu'elles perdent beaucoup plus facilement leur efficacité en protection UV dès lors qu'elles viennent en contact avec de l'eau, cette perte en indice de protection causée par l'élimination progressive à l'eau du filtre hydrophile étant par ailleurs d'autant plus marquée que l'association filtrante lipophile-hydrophile présente dans la composition antisolaire, est synergique au plan de l'indice de protection.

Dans la demande de brevet EP-A- 0 275719, on a cherché à rendre résistantes à l'eau des compositions solaires renfermant des filtres acides, en associant ceux-ci à une amine grasse.

Toutefois, ce type de solution est dans certains cas insatisfaisant du fait de l'impossibilité de combiner certains filtres acides avec des amines grasses et du fait aussi que les amines grasses peuvent provoquer des allergies de contact comme cela est décrit dans l'ouvrage "Adverse reactions to cosmetics" (Anton de Cornelis de Groot - Ed. Rijksuniversiteit Groningen, 1988, chapitre 5, p. 170 et suivantes).

La présente invention vise à résoudre les problèmes ci-dessus.

Plus précisément encore, la présente invention vise à proposer de nouvelles compositions cosmétiques filtrantes qui, tout en mettant en oeuvre un filtre hydrophile comportant au moins un groupement acide dans un support de type émulsion huile-dans-eau, présentent une résistance à l'eau particulièrement améliorée.

Ainsi, à la suite d'importatntes recherches menées sur la question, il a maintenant été trouvé par la Demanderesse que ce but, et d'autres, pouvait être atteint en associant, dans ledit support, ledit filtre hydrophile à groupement acide avec une charge inerte insoluble présentant des propriétés adsorbantes à l'égard dudit filtre.

Cette découverte est la base de la présente invention.

Au sens de la présente invention, la résistance à l'eau (ou rémanence) s'entend de la stabilité au cours du temps de l'indice de protection dans l'UVA et/ou l'UVB d'une composition antisolaire soumise (après application sur la peau ou les cheveux) à des contacts avec de l'eau. On caractérise la protection solaire attachée à une composition donnée en lui affectant un indice de protection (ou IP) qui s'exprime mathématiquement par le rapport du temps d'irradiation nécessaire pour atteindre le seuil érythématogène avec le filtre UV au temps nécessaire pour atteindre le seuil érythématogène sans filtre UV.

La présente invention a ainsi pour premier objet une nouvelle composition cosmétique filtrante, qui est caractérisée par le fait qu'elle comprend, dans un support cosmétiquement acceptable de type émulsion huile-dans-eau, (i) au moins un agent hydrophile capable de filtrer le rayonnement ultraviolet et comportant au moins un radical acide et (ii) au moins une charge insoluble inerte et adsorbante à l'égard dudit agent.

Les compositions antisolaires conformes à l'invention, telles que définies plus en détails ci-après, permettent de conserver des indices de protection suffisants même en cas de mouillage volontaire ou involontaire des parties du corps sur lesquelles elles ont été appliquées. Ceci est non seulement avantageux d'un point de vue économique, mais encore limite ou élimine les risques de "coups de soleil" accidentels pour les utilisateurs qui oublient de se ré-appliquer du produit sur le corps.

Mais d'autres caractéristiques, aspects et avantages de l'invention apparaitront plus complètement encore à la lecture de la description détaillée qui va suivre.

La présente invention vise plus particulièrement, mais non exclusivement, les filtres hydrophiles contenant au moins un radical sulfonique $-SO_3H$. Elle s'applique cependant aussi aux compositions contenant des filtres hydrophiles à radical carboxylique. Le radical acide, carboxylique ou sulfonique, peut, par ailleurs, être sous une forme neutralisée, partiellement ou totalement. Enfin , on notera que, selon l'invention, il est bien entendu possible de mettre en oeuvre un ou plusieurs filtres hydrophiles à fonction acide.

La charge inerte adsorbante qui est utilisée dans le cadre de la présente invention, et qui ne présente pas en tant que telle de caractère photoprotecteur vis à vis des UV, est utile ici uniquement en tant que substance "piége" capable de supporter et de retenir, à sa surface et/ou dans ses pores, le filtre hydrophile comportant au moins un radical acide, et ceci de manière à limiter ou prévenir la solubilisation de ce dernier lorsque la composition antisolaire, une fois appliquée sur la peau et/ou les cheveux, est mise en contact avec de l'eau. Cette substance adsorbante, qui est généralement minérale, présente avantageusement une surface spécifique d'au moins $10 m^2/g$, de préférence d'au moins $50 m^2/g$ et encore plus préférentiellement d'au moins $100 m^2/g$. Il s'agit le plus souvent d'une poudre dont la granulométrie moyenne est d'au moins 0,1 $\mu$m.

Selon un mode de réalisation préférentiel de la présente invention, la charge inerte adsorbante est constituée par une silice (ou dioxyde de silicium), qui peut être pyrogénée ou précipitée ou encore être un gel de silice. De préférence, cette silice est pyrogénée. De telles silices sont notamment vendues sous la dénomination commerciale d'AEROSIL® par la société DEGUSSA ; parmi ces dernières, on préfère plus particulièrement utiliser l'AEROSIL R 972.

D'autres exemples de substances adsorbantes susceptibles de convenir à la présente invention sont les alumines (notamment les alumines actives), les alumino-silicates (argiles notamment), les silicates mixtes de métaux alcalins et/ou alcalino-terreux (smectites, Laponites®, en particulier LAPONITES DS, D, XLS ou XLG commercialisées par la Société LAPORTE Industries, Ltd.), les zéolithes, le talc, la magnésie et autres. On peut, bien entendu, utiliser des mélanges de charges.

On peut par ailleurs faire subir aux charges ci-dessus des traitement de surface spécifiques destinés à les rendre encore plus adsorbantes vis à vis d'un filtre hydrosoluble acide particulier.

Comme exemple de filtres acides contenant au moins un groupe $SO_3H$, on peut plus particulièrement citer les dérivés sulfoniques du 3-benzylidène 2-camphre et notamment ceux de formules (I), (II), (III), (IV), et (V) suivantes :

<u>Formule (I)</u> :

dans laquelle :

- Z désigne un groupement
- n est égal à 0 ou est un nombre entier compris entre 1 et 4 ($0 \leq n \leq 4$),

- $R_1$ représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0, à savoir l'acide benzène 1,4 [di(3-méthylidènecampho 10-sulfonique)].

*Formule (II) :*

dans laquelle :

- $R_2$ désigne un atome d'hydrogène, un atome d'halogène, un radical alkyle contenant de 1 à 4 atomes de carbone environ ou un radical $-SO_3H$,
- $R_3$ et $R_4$ désignent un atome d'hydrogène ou un radical $-SO_3H$, l'un au moins des radicaux $R_2$, $R_3$ ou $R_4$ désignant le radical $-SO_3H$, $R_2$ et $R_4$ ne pouvant désigner simultanément un radical-$SO_3H$.

On peut citer, comme exemples particuliers, les composés suivants de formule (II) dans laquelle :

- $R_2$ désigne le radical $-SO_3H$ en position para du benzylidènecamphre et $R_3$ et $R_4$ désignent chacun un atome d'hydrogène, c'est-à-dire l'acide 4-(3-méthylidènecamphre) benzène sulfonique.
- $R_2$ et $R_4$ désignent chacun un atome d'hydrogène et $R_3$ désigne un radical - $SO_3H$, c'est-à-dire l'acide 3-benzylidène campho-10-sulfonique.
- $R_2$ désigne un radical méthyle en position para du benzylidènecamphre, $R_4$ un radical $-SO_3H$ et $R_3$ un atome d'hydrogène, c'est-à-dire l'acide 2-méthyl 5-(3-méthylidènecamphre) benzène sulfonique.
- $R_2$ désigne un atome de chlore en position para du benzylidènecamphre, $R_4$ un radical $-SO_3H$ et $R_3$ un atome d'hydrogène, c'est-à-dire l'acide 2-chloro 5-(3-méthylidènecamphre) benzène sulfonique.
- $R_2$ désigne un radical méthyle en position para du benzylidènecamphre, $R_4$ désigne un atome d'hydrogène et $R_3$ désigne un radical $-SO_3H$, c'est-à-dire l'acide 3-(4-méthyl) benzylidène campho 10-sulfonique.

*Formule (III) :*

dans laquelle :

- $R_5$ et $R_7$ désignent un atome d'hydrogène, un radical hydroxyle, un radical alkyle ou alcoxy, linéaire ou ramifié, contenant de 1 à 8 atomes de carbone environ, l'un au moins des radicaux $R_5$ et $R_7$ représentant un radical hydroxyle, alkyle ou alcoxy,
- $R_6$ et $R_8$ désignent un atome d'hydrogène, un radical hydroxyle, l'un au moins des radicaux $R_6$ et $R_8$ désignant le radical hydroxyle, sous réserve que lorsque $R_5$ et $R_8$ désignent un atome d'hydrogène et que $R_6$ désigne un radical hydroxyle, $R_7$ ne désigne pas un radical alcoxy ou un atome d'hydrogène.

On peut citer, comme exemples particuliers, les composés suivants de formule (III) dans laquelle :

- $R_5$ est un radical méthyle, $R_6$ un atome d'hydrogène, $R_7$ un radical tertiobutyle, $R_8$ un radical hydroxyle, c'est-à-dire l'acide (3-t-butyl 2-hydroxy 5-méthyl) benzylidène campho-10-sulfonique.
- $R_5$ est un radical méthoxy, $R_6$ un atome d'hydrogène, $R_7$ un radical tertiobutyle, $R_8$ un radical hydroxyle, c'est-à-dire l'acide (3-t-butyl 2-hydroxy 5-méthoxy) benzylidène campho-10-sulfonique.
- $R_5$ et $R_7$ désignent chacun un radical tertiobutyle, $R_6$ un radical hydroxyle, $R_8$ un atome d'hydrogène, c'est-à-dire l'acide (3,5-diterbutyl 4-hydroxy) benzylidène campho-10-sulfonique.

*Formule (IV) :*

dans laquelle :

- $R_9$ désigne un atome d'hydrogène, un radical alkyle, linéaire ou ramifié, contenant de 1 à 18 atomes de carbone environ, un radical alcényle, linéaire ou ramifié, contenant de 3 à 18 atomes de carbone environ, un groupement ou $-(CH_2CH_2O)_n-H$, ou $-CH_2-CHOH-CH_3$ ou encore un radical divalent : $-(CH_2)_m-$ ou $-CH_2-CHOH-CH_2-$, n étant un nombre entier compris entre 1 et 6 ($1 \leq n \leq 6$) et m un nombre entier compris entre 1 et 10 ($1 \leq m \leq 10$),

- $R_{10}$ désigne un atome d'hydrogène, un radical alcoxy contenant de 1 à 4 atomes de carbone environ ou un radical divalent - O - relié au radical $R_9$ lorsque celui-ci est divalent lui aussi,

- q désigne un nombre entier égal à 1 ou 2, étant entendu que si q est égal à 2, $R_9$ doit désigner un radical divalent,

- Y et Y' désignent un atome d'hydrogène ou un radical $-SO_3H$, au moins un de ces radicaux Y ou Y' étant différent de l'hydrogène.

On peut citer, comme exemples particuliers, les composés suivants de formule (IV) dans laquelle :

- q est égal à 1, Y et $R_{10}$ désignent chacun un atome d'hydrogène, $R_9$ désigne un radical méthyle, Y' en position 3 désigne un radical $-SO_3H$, c'est-à-dire l'acide 2-méthoxy 5-(3-méthylidènecamphre) benzène sulfonique.

- q est égal à 1, Y désigne un radical $-SO_3H$, Y' un atome d'hydrogène, $R_{10}$ un radical divalent -O- relié à $R_9$ désignant un radical méthylène, c'est-à-dire l'acide 3-(4,5-méthylènedioxy) benzylidène campho-10-sulfonique.

- q est égal à 1, Y désigne un radical $-SO_3H$, Y' et $R_{10}$ désignent tous deux un atome d'hydrogène, $R_9$ désigne un radical méthyle, c'est-à-dire l'acide 3-(4-méthoxy) benzylidène campho-10-sulfonique.

- q est égal à 1, Y désigne un radical $-SO_3H$, Y' un atome d'hydrogène, $R_9$ désigne un radical méthyle, $R_{10}$ désigne un radical méthoxy, c'est-à-dire l'acide 3-(4,5-diméthoxy) benzylidène campho-10-sulfonique.

- q est égal à 1, Y désigne un radical $-SO_3H$, Y' et $R_{10}$ désignent tous deux un atome d'hydrogène, et $R_9$ un radical n butyle, c'est-à-dire l'acide 3-(4-n.butoxy) benzylidène campho-10-sulfonique.

- q est égal à 1, Y désigne un radical $-SO_3H$, Y' un atome d'hydrogène, $R_9$ désigne un radical n butyle, $R_{10}$ un radical méthoxy, c'est-à-dire l'acide 3-(4-n.butoxy 5-méthoxy) benzylidène campho-10-sulfonique.

_Formule (V)_ :

dans laquelle :

- $R_{11}$ désigne un atome d'hydrogène, un radical alkyle ou alcoxy, linéaire ou ramifié, contenant de 1 à 6 atomes de carbone environ ou un radical - $SO_3H$,

- $R_{12}$ désigne un atome d'hydrogène, un radical alkyle ou alcoxy, linéaire ou ramifié, contenant de 1 à 6 atomes de carbone environ,

- $R_{13}$ désigne un atome d'hydrogène ou un radical $-SO_3H$, l'un au moins des radicaux $R_{11}$ et $R_{13}$ désignant un radical $-SO_3H$,

- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer, comme exemple particulier de formule (V), le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène) phényl] benzimidazole-5-sulfonique.

Les composés de structures (I), (II), (III), (IV), (V) ci-dessus sont respectivement décrits dans le brevet US 4 585 597 et les demandes de brevets FR 2 236 515, 2 282 426, 2 645 148, 2 430 938 et 2 592 380.

Le filtre à groupement sulfonique peut également être un dérivé sulfonique de benzophénone de formule (VI) suivante : dans laquelle :

- $R_{14}$ et $R_{15}$, identiques ou différents, désignent soit un atome d'hydrogène soit un radical alkyle, linéaire ou ramifié,

contenant de 1 à 8 atomes de carbone environ,

- a, b et c , identiques ou différents, sont des nombres égaux à 0 ou 1.

On peut citer comme exemple particulier de composé de formule (VI) : l'acide 2-hydroxy 4-méthoxybenzophénone 5-sulfonique (composé de formule (VI) dans laquelle a, b, et c sont égaux à zéro, et $R_{15}$ désigne un radical méthyle).

Le filtre à groupement sulfonique peut encore être un dérivé sulfonique de formule (VII) suivante : dans laquelle :

- X désigne un atome d'oxygène ou un radical -NH-,
- $R_{16}$ désigne un atome d'hydrogène, un radical alkyle ou alcoxy, linéaire ou ramifié, contenant de 1 à 8 atomes de carbone environ ou un groupement de formule (VIII) dans laquelle X' représente un atome d'oxygène ou un radical -NH-.

On peut citer, comme exemples particuliers, les composés suivants de formule (VII) dans laquelle :

- X désigne le radical -NH- et $R_{16}$ désigne un atome d'hydrogène : l'acide 2-phénylbenzimidazole 5-sulfonique.
- X désigne le radical -NH-, $R_{16}$ désigne le groupement de formule (VIII) dans lequel X' désigne le radical -NH- : l'acide benzène 1,4 -di(benzimidazol - 2 yl-5-sulfonique).
- X désigne un atome d'oxygène, $R_{16}$ désigne le groupement de formule (VIII) dans lequel X' désigne un atome d'oxygène : l'acide benzène 1,4-di(benzoxazol-2 yl -5-sulfonique).

Les composés de formule (VI) et (VII) sont des composés connus pouvant être préparés selon des méthodes classiques décrites dans l'art antérieur.

Des exemples de compositions cosmétiques filtrantes préférées dans le cadre de la présente invention comprennent les associations entre filtre UV hydrophile acide et charge inerte adsorbante suivantes :

- dérivé sulfonique de 3-benzylidène 2-camphre de formule (I) dans laquelle n = 0 (acide benzène 1,4 (di(3-méthylidène campho-10-sulfonique)) et silice pyrogénée de type AEROSIL R 972 vendue par la société DEGUSSA.
- dérivé sulfonique de benzophénone de formule (IV) dans laquelle a, b et c sont égaux à 0, et $R_{15}$ désigne un radical méthyle (acide 2-hydroxy 4-méthoxybenzophénone 5- sulfonique, notamment vendu par BASF sous le nom de Uvinul MS 40 ) et silice pyrogénée de type AEROSIL R 972 vendue par la société DEGUSSA.
- dérivé sulfonique de benzimidazole de formule (VII) dans laquelle X désigne le radical -NH- et $R_{16}$ désigne un atome d'hydrogène (acide 2-phénylbenzimidazole 5-sulfonique, notamment vendu par MERCK sous le nom de Eusolex 232) et silice pyrogénée de type AEROSIL R 972 vendue par la société DEGUSSA.

Le filtre UV hydrophile comportant au moins un radical acide, et notamment sulfonique, est généralement présent dans les compositions filtrantes selon l'invention à une concentration totale comprise entre 0,2 et 10 % en poids environ, et de préférence entre 0,25 et 6 % en poids environ, par rapport au poids total de la composition.

La charge inerte adsorbante, et notamment la silice, est de préférence présente dans les compositions filtrantes en une teneur comprise entre 0,15 et 5 % environ, et de préférence entre 0,2 et 3 % environ, par rapport au poids total de la composition.

Les compositions cosmétiques selon l'invention peuvent bien entendu contenir un ou plusieurs filtres solaires complémentaires actifs dans l'UVA et/ou l'UVB, hydrophiles ou lipophiles autres, bien sûr, que les filtres hydrophiles acides de l'invention.

Ces filtres complémentaires sont choisis de préférence parmi les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre ou l'acide camphosulfonique (1,4 divinylbenzène), les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, les dérivés de l'acide p-aminobenzoïque comme par exemple le para-diméthylaminobenzoate d'octyle, l'anthranilate de menthyle, les polymères filtres et silicones filtres décrits dans la demande WO-93/04665. D'autres exemples de filtres organiques sont donnés dans la demande de brevet EP-A 0 487

404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane, de fer, de zinc, de zirconium ou de cérium qui sont des agents photoprotecteurs bien connus en soi agissant par blocage physique (réflection et/ou diffusion) du rayonnement UV. De tels nanopigments d'oxydes métalliques, enrobés ou non enrobés, sont en particulier décrits dans les demande de brevets EP-A- 0 518 772 et EP-A-0 518 773. On peut par ailleurs mentionner, à titre complémentaire, les produits vendus sous les noms de marque UVT M 160, UVT M 212 et UVT M 262 par la Société KEMIRA, et MT 100 SAS par la Société TAYCA.

Les compositions de l'invention peuvent comprendre en outre des adjuvants cosmétiques choisis parmi les corps gras, les solvants organiques, les épaississants non ioniques, les adoucissants, les antioxydants, les opacifiants, les stabilisants, les silicones , les agents anti-mousse, les agents hydratants, les vitamines, les parfums, les conservateurs, les tensioactifs non ioniques, les charges, les séquestrants, les polymères non ioniques, les propulseurs, les agents alcalinisants ou acidifiants, les colorants, ou tout autre ingrédient habituellement utilisé en cosmétique, en particulier pour la fabrication de compositions sous forme d'émulsions.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en $C_{12}$-$C_{15}$ (Finsolv TN de FINETEX), l'alcool myristique polyoxypropyléné à 3 moles d'oxyde de propylène (WITCONOL APM de WITCO), les triglycérides d'acides gras en $C_6$-$C_{18}$ tels que les triglycérides d'acide caprylique/caprique, les huiles fluorées et perfluorées.

Les huiles peuvent être choisies parmi les huiles animales, végétales, minérales ou de synthèse et notamment l'huile de palme hydrogénée, l'huile de ricin hydrogénée ou non, l'huile de vaseline, l'huile de paraffine, l'huile de Purcellin (octanoate de stéaryle), les huiles de silicone, volatiles ou non, les isoparaffines et les poly-$\alpha$-oléfines.

Les cires peuvent être choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Parmi les solvants organiques, on peut citer les alcools et polyols inférieurs tels que l'éthanol, l'isopropanol, le propylèneglycol, la glycérine et le sorbitol.

Les épaississants peuvent être choisis notamment parmi les acides polyacryliques réticulés, les gommes de guar et celluloses modifiées ou non telles que la gomme de guar hydroxypropylée, la cétylhydroxyéthylcellulose et l'hydroxypropylméthyl cellulose.

Les compositions de l'invention sont préparées selon les techniques bien connues de l'homme de l'art dans le domaine de la synthèse des émulsions de type huile-dans-eau. Lors de la préparation de la composition, on introduit le filtre acide préalablement neutralisé, par exemple, au moyen de triéthanolamine, à la phase grasse de la composition, puis on ajoute la charge inerte adsorbante, par exemple de la silice, à cette phase grasse jusqu'à l'obtention d'un liquide visqueux ; on additionne ensuite les autres constituants de la composition et on homogénéise.

A titre indicatif, dans les émulsions antisolaires conformes à l'invention destinées à la protection de la peau, la phase aqueuse est présente généralement à raison de 50 à 95 % en poids environ, de préférence de 70 à 90 % en poids environ, par rapport à l'ensemble de la formulation, la phase huileuse à raison de 5 à 50 % en poids environ, de préférence de 10 à 30 % en poids environ, par rapport à l'ensemble de la formulation, et le ou les (co)émulsionnant(s) à raison de 0,5 à 20 % en poids environ, de préférence de 2 à 10% en poids environ, par rapport à l'ensemble de la formulation.

La composition cosmétique de l'invention peut être utilisée comme composition protectrice de l'épiderme humain ou des cheveux contre les rayons ultraviolets, comme composition antisolaire ou comme produit de maquillage (des cils, des sourcils ou de la peau).

Cette composition peut se présenter, selon l'application envisagée, par exemple sous la forme d'une crème, d'un lait, d'un gel crème, de pommade, de shampooing ou de toute autre composition capillaire à rincer destinée à être appliquée avant ou après shampooing, avant, pendant ou après coloration ou décoloration, avant, pendant ou après permanente ou défrisage, et éventuellement enfin être conditionnée en aérosol et se présenter alors sous forme de mousse ou de spray.

La phase aqueuse de l'émulsion peut comprendre une dispersion vésiculaire non ionique préparée selon des procédés connus (Bangham, Standish and Watkins. J. Mol. Biol. 13, 238 (1965), FR 2 315 991 et FR 2 416 008).

Un autre objet de la présente invention est un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

**EXEMPLE 1**

*Lait solaire pour la peau* (émulsion huile-dans-eau)

| | |
|---|---|
| - Mélange d'alcool cétylstéarylique et d'alcool cétylstéarylique oxyéthyléné à 33 moles d'oxyde d'éthylène vendu sous la dénomination "Sinnowax AO" par HENKEL | 7 g |
| - Mélange de mono et distéarate de glycérol non autoémulsifiable | 2 g |
| - Alcool cétylique | 1,5 g |
| - Benzoate d'alcools en C12-C15 (Finsolv TN de FINETEX) | 10 g |
| - Huile de silicone (Silbione Huile 70 047 V 300 de RHONE POULENC SILICONES) | 1,5 g |
| - 4-Tert-butyl 4'-méthoxy dibenzoylméthane | 4 g |
| - 2-cyano, 3,3-diphénylacrylate de 2-éthyl hexyle | 10 g |
| - Silice pyrogénée (Aerosil R 972 de DEGUSSA) | 0,5g |
| - Acide 2-phényl benzimidazole 5-sulfonique (Eusolex 232 de MERCK) | 3 g |
| - Triéthanolamine | 2,21g |
| - Glycérine | 5 g |
| - Parfums, Conservateurs qs | |
| - Eau déminéralisée qsp | 100 g |

On réalise l'émulsion ci-dessus en additionnant la phase grasse portée aux environs de 80°C à la phase aqueuse renfermant la glycérine et les conservateurs, portée à la même température et sous agitation rapide.

**EXEMPLE 2**

*Crème solaire pour la peau* (émulsion huile-dans-eau)

| | |
|---|---|
| - Mélange d'alcool cétylstéarylique et d'alcool cétylstéarylique oxyéthyléné à 33 moles d'oxyde d'éthylène vendu sous la dénomination "Sinnowax AO" par HENKEL | 7 g |
| - Mélange de mono et distéarate de glycérol non autoémulsifiable | 2 g |
| - Alcool cétylique | 1,5 g |
| - Benzoate d'alcools en C12-C15 (Finsolv TN de FINETEX) | 10 g |
| - Huile de silicone (Silbione Huile 70 047 V 300 de RHONE POULENC SILICONES) | 1,5 g |
| - 4-Tert-butyl 4'-méthoxy dibenzoylméthane | 2 g |
| - 2-cyano, 3,3-diphénylacrylate de 2-éthyl hexyle | 10 g |
| - Silice pyrogénée (Aerosil R 972 de DEGUSSA) | 0,4g |
| - Acide benzène 1,4 (di(3-méthylidènecampho-10-sulfonique)] | 1,98g |

(suite)

| | |
|---|---|
| - Triéthanolamine | 1,84g |
| - Glycérine | 5g |
| - Parfums, Conservateurs qs | |
| - Eau déminéralisée qsp | 100 g |

On réalise l'émulsion ci-dessus en additionnant la phase grasse portée aux environs de 80°C à la phase aqueuse renfermant la glycérine et les conservateurs, portée à la même température et sous agitation rapide.

**EXEMPLE 3**

*Crème solaire pour la peau* (émulsion huile-dans-eau)

| | |
|---|---|
| - Mélange d'alcool cétylstéarylique et d'alcool cétylstéarylique oxyéthyléné à 33 moles d'oxyde d'éthylène vendu sous la dénomination "Sinnowax AO" par HENKEL | 7 g |
| - Mélange de mono et distéarate de glycérol non autoémulsifiable | 2 g |
| - Alcool cétylique | 1,5 g |
| - Benzoate d'alcools en C12-C15 (Finsolv TN de FINETEX) | 10 g |
| - Huile de silicone (Silbione Huile 70 047 V 300 de RHONE POULENC SILICONES) | 1,5 g |
| - 4-Tert-butyl 4'-méthoxy dibenzoylméthane | 4g |
| - 2-cyano, 3,3-diphénylacrylate de 2-éthyl hexyle | 10 g |
| - Silice pyrogénée (Aerosil R 972 de DEGUSSA) | 0,6g |
| - Acide benzène 1,4 (di(3-méthylidènecampho-10-sulfonique)] | 2,97g |
| - Triéthanolamine | 2,31g |
| - Glycérine | 5 g |
| - Nanopigment d'oxyde de titane enrobé de stéarate d'aluminium et d'alumine (Micro Titanium Dioxyde MT100Tde la société TAYCA) | 5g |
| - Parfums, Conservateurs qs | |
| - Eau déminéralisée qsp | 100 g |

On réalise l'émulsion ci-dessus en additionnant la phase grasse portée aux environs de 80°C à la phase aqueuse renfermant la glycérine, le nanopigment d'oxyde de titane et les conservateurs, portée à la même température et sous agitation rapide.

**EXEMPLE 4**

*Lait solaire pour la peau* (émulsion huile-dans-eau)

| | |
|---|---|
| - Mélange d'alcool cétylstéarylique et d'alcool cétylstéarylique oxyéthyléné à 33 moles d'oxyde d'éthylène vendu sous la dénomination "Sinnowax AO" par HENKEL | 7 g |
| - Mélange de mono et distéarate de glycérol non autoémulsifiable | 2 g |
| - Alcool cétylique | 1,5 g |
| - Benzoate d'alcools en C12-C15 (Finsolv TN de FINETEX) | 10 g |

(suite)

| | |
|---|---|
| - Huile de silicone (Silbione Huile 70 047 V 300 de RHONE POULENC SILICONES) | 1,5 g |
| - 4-Tert-butyl 4'-méthoxy dibenzoylméthane | 1,5g |
| - 3-(4-Méthyl benzylidène) camphre (Eusolex 6300 de MERCK) | 4g |
| - Silice pyrogénée (Aerosil R 972 de DEGUSSA) | 0,5g |
| - Acide benzène 1,4 (di(3-méthylidènecampho-10-sulfonique)] | 1,98g |
| - Triéthanolamine | 1,44g |
| - Glycérine | 5g |
| - Nanopigment d'oxyde de titane enrobé de stéarate d'aluminium et d'alumine (Micro Titanium Dioxyde MT 100Tde la société TAYCA) | 5g |
| - Parfums, Conservateurs qs | |
| - Eau déminéralisée qsp | 100 g |

On réalise l'émulsion ci-dessus en additionnant la phase grasse portée aux environs de 80°C à la phase aqueuse renfermant la glycérine, le nanopigment d'oxyde de titane et les conservateurs, portée à la même température et sous agitation rapide.

**EXEMPLE 5**

*Lait solaire pour la peau* (émulsion huile-dans-eau)

| | |
|---|---|
| - Mélange d'alcool cétylstéarylique et d'alcool cétylstéarylique oxyéthyléné à 33 moles d'oxyde d'éthylène vendu sous la dénomination "Sinnowax AO" par HENKEL | 7 g |
| - Mélange de mono et distéarate de glycérol non autoémulsifiable | 2 g |
| - Alcool cétylique | 1,5 g |
| - Benzoate d'alcools en C12-C15 (Finsolv TN de FINETEX) | 10 g |
| - Huile de silicone (Silbione Huile 70 047 V 300 de RHONE POULENC SILICONES) | 1,5 g |
| - p-méthoxycinnamate de 2-éthylhexyle | 7 g |
| - 2-cyano 3,3-diphénylacrylate de 2-éthylhexyle- | 7 g |
| - acide $\beta,\beta$' camphosulfonique [1,4 divinylbenzène] en solution aqueuse à 33% | 9,09 g |
| - Smectite synthétique | 2 g |
| - Triéthanolamine | 1,44g |
| - Glycérine | 5g |
| - Nanopigment d'oxyde de titane enrobé de stéarate d'aluminium et d'alumine (Micro Titanium Dioxyde MT 100Tde la société TAYCA) | 3 g |
| - Parfums, Conservateurs qs | |
| - Eau déminéralisée qsp | 100 g |

On réalise l'émulsion ci-dessus en additionnant la phase grasse portée aux environs de 80°C à la phase aqueuse renfermant la glycérine, le nanopigment d'oxyde de titane et les conservateurs, portée à la même température et sous agitation rapide.

## Revendications

1. Composition cosmétique filtrante, caractérisée par le fait qu'elle contient, dans un support cosmétiquement acceptable de type émulsion huile-dans-eau, (i) au moins un agent hydrophile filtrant le rayonnement ultraviolet et comportant au moins un radical acide, éventuellement neutralisé, et (ii) au moins une charge insoluble inerte et adsorbante à l'égard dudit agent.

2. Composition selon la revendication 1, caractérisée en ce que le radical acide de l'agent hydrophile filtrant le rayonnement ultraviolet est un radical sulfonique $-SO_3H$.

3. Composition selon les revendications 1 ou 2, caractérisée en ce que l'agent hydrophile filtrant le rayonnement ultraviolet est un composé de structure 3-benzylidène 2-camphre.

4. Composition selon la revendication 3, caractérisé en ce que l'agent hydrophile filtrant le rayonnement ultraviolet répond à la formule (I) suivante :

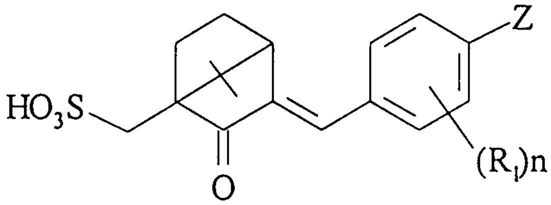

(I)

dans laquelle :

- Z désigne un groupement :

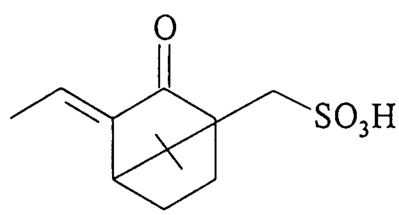

- n désigne 0 ou un nombre entier supérieur ou égal à 1 et inférieur ou égal à 4,
- $R_1$ représente un ou plusieurs radicaux alkyle ou alkoxy, identiques ou différents, linéaires ou ramifiés, contenant de 1 à 4 atomes de carbone.

5. Composition selon la revendication 4, caractérisée en ce que le composé de formule (I) est l'acide benzène 1,4 [di(3-méthylidènecampho-10-sulfonique)].

6. Composition selon la revendication 3, caractérisée en ce que l'agent hydrophile filtrant le rayonnement ultraviolet répond à la formule (II) suivante :

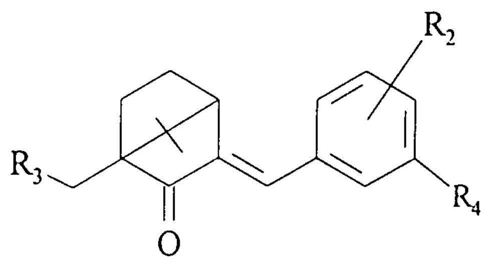

(II)

dans laquelle

- $R_2$ désigne un atome d'hydrogène, un atome d'halogène, un radical alkyle contenant 1 à 4 atomes de carbone, un radical $-SO_3H$,

- $R_3$ et $R_4$ désignent un atome d'hydrogène ou un radical $-SO_3H$, l'un au moins des radicaux $R_2$, $R_3$ ou $R_4$ désignant le radical $-SO_3H$, $R_2$ et $R_4$ ne pouvant désigner simultanément un radical-$SO_3H$.

**7.** Composition selon la revendication 6, caractérisée en ce que dans la formule (II) $R_2$ désigne le radical $-SO_3H$ en position para du benzylidène camphre et $R_3$ et $R_4$ représentent chacun un atome d'hydrogène.

**8.** Composition selon la revendication 6, caractérisée en ce que dans la formule (II) $R_2$ et $R_4$ représente chacun un atome d'hydrogène et $R_3$ désigne $-SO_3H$.

**9.** Composition selon la revendication 6, caractérisée en ce que dans la formule (II) $R_2$ désigne le radical méthyle en position para du benzylidène camphre, $R_4$ un radical représente le radical $SO_3H$ et $R_3$ un atome d'hydrogène.

**10.** Composition selon la revendication 6, caractérisée en ce que dans la formule (II) $R_2$ désigne un atome de chlore en position para du benzylidène camphre, $R_4$ le radical $-SO_3H$ et $R_3$ un atome d'hydrogène.

**11.** Composition selon la revendication 6, caractérisée en ce que dans la formule (II) $R_2$ désigne le radical méthyle en position para du benzylidène camphre, $R_4$ désigne un atome d'hydrogène et $R_3$ désigne le radical $-SO_3H$.

**12.** Composition selon la revendication 3, caractérisée en ce que l'agent hydrophile filtrant le rayonnement ultraviolet répond à la formule (III) suivante :

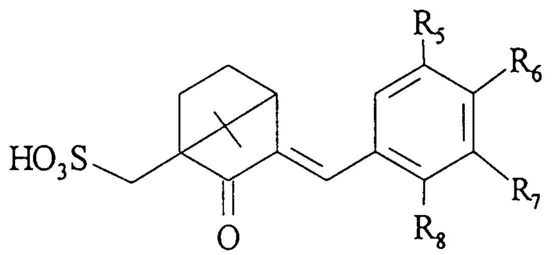

(III)

dans laquelle :

- $R_5$ et $R_7$, identiques ou différents, désignent un atome d'hydrogène, un radical hydroxyle, un radical alkyle linéaire ou ramifié contenant 1 à 8 atomes de carbone ou un radical alcoxy linéaire ou ramifié contenant 1 à 8 atomes de carbone, l'un au moins des radicaux $R_5$ et $R_7$ représentant un radical hydroxyle, alkyle ou alcoxy,

- $R_6$ et $R_8$, identiques ou différents, désignent un atome d'hydrogène, un radical hydroxyle, l'un au moins des radicaux $R_6$ et $R_8$ désignant le radical hydroxyle,

- sous réserve que lorsque $R_5$ et $R_8$ désignent l'hydrogène et que $R_6$ désigne le radical hydroxyle, $R_7$ ne désigne

pas un radical alcoxy ou un atome d'hydrogène.

**13.** Composition selon la revendication 12, caractérisée en ce que dans la formule (III) $R_5$ est un radical méthyle, $R_6$ un atome d'hydrogène, $R_7$ un radical tertiobutyle et $R_8$ un radical hydroxyle.

**14.** Composition selon la revendication 12, caractérisée en ce que dans la formule (III) $R_5$ est un radical méthoxy, $R_6$ un atome d'hydrogène, $R_7$ un radical tertiobutyle et $R_8$ un radical hydroxyle.

**15.** Composition selon la revendication 12, caractérisée en ce que dans la formule (III) $R_5$ et $R_7$ désignent chacun un radical tertiobutyle, $R_6$ un radical hydroxyle et $R_8$ un atome d'hydrogène.

**16.** Composition selon la revendication 3, caractérisée en ce que l'agent hydrophile filtrant le rayonnement ultraviolet répond à la formule (IV) suivante :

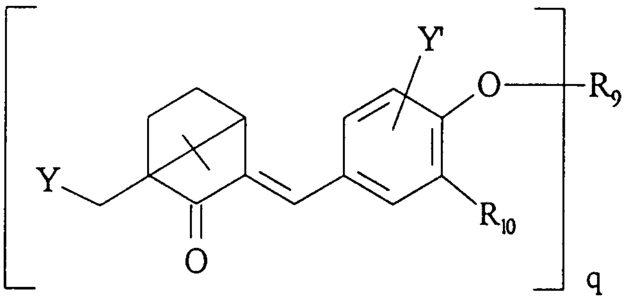

(I V)

dans laquelle

- $R_9$ désigne un atome d'hydrogène, un radical alkyle linéaire ou ramifié contenant de 1 à 18 atomes de carbone , un radical alcényle, linéaire ou ramifié, contenant de 3 à 18 atomes de carbone, un groupement choisi parmi :

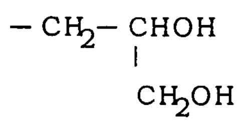

,

ou $-(CH_2CH_2O)_n-H$, ou $-CH_2-CHOH-CH_3$, ou $-(CH_2)_m-$ ou $-CH_2-CHOH-CH_2-$, n étant un nombre entier compris entre 1 et 6 ($1 \leq n \leq 6$) et m un nombre entier compris entre 1 et 10 ($1 \leq m \leq 10$),

- $R_{10}$ désigne un atome d'hydrogène, un radical alcoxy contenant de 1 à 4 atomes de carbone, ou un radical divalent - O - relié au radical $R_9$ lorsque celui-ci est divalent lui aussi.

- q désigne un nombre entier égal à 1 ou 2, étant entendu que si q est égal à 2, $R_9$ doit désigner un radical divalent.

- Y et Y' désignent un atome d'hydrogène ou un radical $-SO_3H$, au moins un de ces radicaux Y ou Y' est différent de l'hydrogène.

**17.** Composition selon la revendication 16, caractérisée en ce que dans la formule (IV) q est égal à 1, Y et $R_{10}$ désignent chacun un atome d'hydrogène, $R_9$ désigne un radical méthyle, Y' en position 3 désigne un radical $-SO_3H$.

**18.** Composition selon la revendication 16, caractérisée en ce que dans la formule (IV) q est égal à 1, Y désigne un radical $-SO_3H$, Y' un atome d'hydrogène, $R_{10}$ un radical divalent -O- relié à $R_9$ désignant un radical méthylène.

**19.** Composition selon la revendication 16, caractérisée en ce que dans la formule (IV) q est égal à 1, Y désigne un radical $-SO_3H$, Y' et $R_{10}$ désignent tous deux un atome d'hydrogène, et $R_9$ désigne un radical méthyle.

**20.** Composition selon la revendication 16, caractérisée en ce que dans la formule (IV) q est égal à 1, Y désigne un radical $-SO_3H$, Y' un atome d'hydrogène, $R_9$ désigne un radical méthyle et $R_{10}$ désigne un radical méthoxy.

**21.** Composition selon la revendication 16, caractérisée en ce que dans la formule (IV) q est égal à 1, Y désigne un radical $-SO_3H$, Y' et $R_{10}$ désignent tous deux un atome d'hydrogène, et $R_9$ un radical n butyle.

**22.** Composition selon la revendication 16, caractérisée en ce que dans la formule (IV) q est égal à 1, Y désigne un radical $-SO_3H$, Y' un atome d'hydrogène, $R_9$ désigne un radical n butyle et $R_{10}$ un radical méthoxy.

**23.** Composition selon la revendication 3, caractérisée en ce que l'agent hydrophile filtrant le rayonnement ultraviolet répond à la formule (V) suivante :

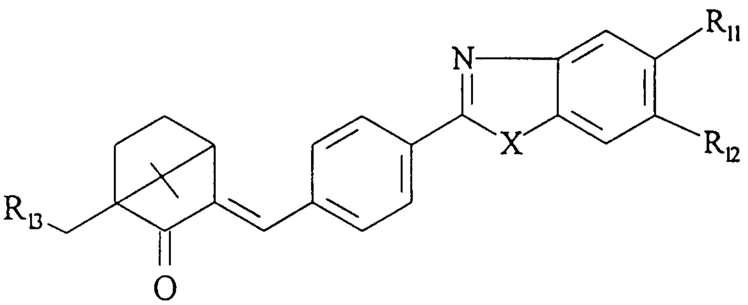

(V)

dans laquelle :

- $R_{11}$ désigne un atome d'hydrogène, un radical alkyle ou alcoxy, linéaire ou ramifié, contenant 1 à 6 atomes de carbone, un radical $-SO_3H$,
- $R_{12}$ désigne un atome d'hydrogène, un radical alkyle ou alcoxy linéaire ou ramifié contenant 1 à 6 atomes de carbone,
- $R_{13}$ désigne un atome d'hydrogène, ou un radical $-SO_3H$,
- l'un au moins des radicaux $R_{11}$ ou $R_{13}$ désigne un radical $-SO_3H$,
- X est un atome d'oxygène ou de soufre, ou un groupement -NR-, R étant un atome d'hydrogène ou un radical alkyle linéaire ou ramifié contenant de 1 à 6 atomes de carbone.

**24.** Composition selon la revendication 23, caractérisée en ce que dans la formule (V) X désigne un radical -NH-, $R_{11}$désigne un radical $-SO_3H$, $R_{12}$ et $R_{13}$ désignent tous deux un atome d'hydrogène.

**25.** Composition selon les revendications 1 ou 2, caractérisée en ce que l'agent hydrophile filtrant le rayonnement ultraviolet est un composé de formule (VI)

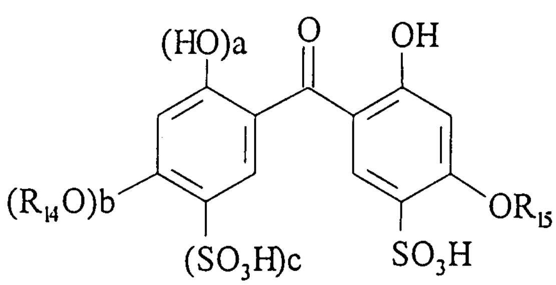

(VI)

dans laquelle

- $R_{14}$ et $R_{15}$ identiques ou différents, désignent un atome d'hydrogène ou un radical alkyle, linéaire ou ramifié, contenant de 1 à 8 atomes de carbone,

- a, b et c identiques ou différents sont égaux à 0 ou 1.

**26.** Composition selon la revendication 25, caractérisée en ce que dans la formule (VI) a=b=c=0 et $R_{15}$ désigne un radical méthyle.

**27.** Composition selon les revendications 1 ou 2, caractérisée par le fait que l'agent hydrophile filtrant le rayonnement ultraviolet est un composé de formule (VII)

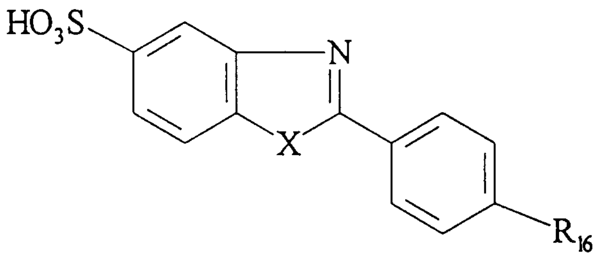

(VII)

dans laquelle :

- X désigne un atome d'oxygène ou un radical - NH -,

- $R_{16}$ désigne un atome d'hydrogène, un radical alkyle ou alcoxy, linéaire ou ramifié, contenant de 1 à 8 atomes de carbone ou un groupement de formule (VIII)

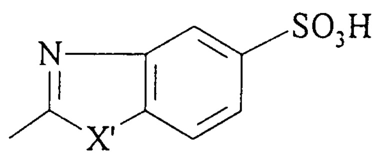

(VIII)

dans laquelle X' désigne indépendamment de X, un atome oxygène ou un radical -NH-.

**28.** Composition selon la revendication 27, caractérisée en ce que dans la formule (VII) X désigne le radical -NH-, et $R_{16}$ désigne un atome d'hydrogène.

**29.** Composition selon la revendication 27, caractérisée en ce que dans la formule (VII) X désigne le radical -NH-, $R_{16}$ désigne le groupement de formule (VIII) avec X' désignant le radical -NH-.

**30.** Composition selon la revendication 27, caractérisée en ce que dans la formule (VII) X désigne un atome d'oxygène, $R_{16}$ désigne le groupement de formule (VIII) avec X' désignant un atome d'oxygène.

**31.** Composition selon l'une quelconque des revendications précédentes, caractérisée en ce que la surface spécifique de ladite charge inerte adsorbante est d'au moins 10 $m^2/g$, de préférence d'au moins 50 $m^2/g$ et encore plus préférentiellement d'au moins 100 $m^2/g$.

**32.** Composition selon l'une quelconque des revendications précédentes, caractérisée en ce que ladite charge inerte adsorbante est choisie au sein du groupe constitué par les silices, les alumines, les alumino-silicates les zéolithes, le talc et la magnésie.

**33.** Composition selon la revendication 32, caractérisée en ce que la charge inerte adsorbante est une silice adsorbante pyrogénée, précipitée ou un gel de silice.

**34.** Composition selon l'une quelconque des revendications précédentes caractérisée par le fait qu'elle renferme de 0,2 à 10 % en poids environ d'agent hydrophile filtrant le rayonnement ultraviolet, et de préférence 0,25 à 6 % en poids environ, par rapport au poids total de la composition.

**35.** Composition selon l'une quelconque des revendications précédentes, caractérisée par le fait qu'elle renferme la charge inerte adsorbante en une proportion comprise entre 0,15 et 5 % en poids environ, et de préférence entre 0,2 et 3 % en poids environ, par rapport au poids total de la composition.

**36.** Composition selon l'une quelconque des revendications précédentes, caractérisée par le fait qu'elle contient en outre un ou plusieurs filtres solaires complémentaires actifs dans l'UVB et/ou l'UVA hydrophiles ou lipophiles autres que les filtres hydrophiles acides selon la revendication 1.

**37.** Composition selon la revendication 36, caractérisée par le fait que les filtres solaires complémentaires sont choisis parmi les cinnamates, les salicylates, les dérivés du benzylidène camphre, les dérivés de triazine, les dérivés de la benzophénone, les dérivés du dibenzoylméthane, les dérivés de β,β-diphénylacrylate, les dérivés de l'acide p-aminobenzoïque, l'anthranilate de menthyle, les polymères filtres et les silicones filtres.

**38.** Composition selon la revendication 37, caractérisée par le fait que les filtres solaires complémentaires sont choisis parmi le groupe comprenant le 4-tert-butyl-4'-méthoxydibenzoylméthane, le 2-cyano 3,3-diphénylacrylate de 2-éthyl hexyle et le 3-(4-méthylbenzylidène)-camphre.

**39.** Composition selon l'une quelconque des revendications précédentes, caractérisée en ce qu'elle contient en outre, à titre d'agents photoprotecteurs complémentaires, des nanopigments d'oxydes métalliques, enrobés ou non, capables de bloquer physiquement, par diffusion et/ou réflection, le rayonnement UV.

**40.** Composition selon l'une quelconque des revendications précédentes, caractérisée par le fait qu'elle comprend en outre des adjuvants cosmétiques choisis parmi les corps gras, les solvants organiques, les épaississants non ioniques, les adoucissants, les antioxydants, les opacifiants, les stabilisants, les silicones, les agents anti-mousse, les agents hydratants, les parfums, les conservateurs, les tensioactifs non ioniques, les charges, les séquestrants, les polymères non ioniques, les propulseurs, les agents alcalinisants ou acidifiants, les colorants.

**41.** Composition selon l'une quelconque des revendications précédentes, caractérisée en ce qu'elle constitue une composition protectrice de l'épiderme humain ou une composition antisolaire.

**42.** Composition selon l'une quelconque des revendications 1 à 40, caractérisée en ce qu'elle constitue une composition de maquillage des cils, des sourcils ou de la peau.

**43.** Composition selon l'une quelconque des revendications 1 à 40, caractérisée en ce qu'elle constitue une composition pour la protection des cheveux contre les rayons ultraviolets.

**44.** Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

**45.** Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 43.

**46.** Utilisation d'une charge inerte insoluble adsorbante, en particulier une silice, pour améliorer la rémanence à l'eau d'une composition cosmétique antisolaire de type émulsion huile-dans-eau contenant, à titre d'agent photoprotecteur, un filtre UV hydrophile comportant au moins un radical acide, en particulier sulfonique.

## Claims

**1.** Cosmetic screening composition, characterized in that it contains, in a cosmetically acceptable vehicle of the oil-in-water emulsion type, (i) at least one hydrophilic agent which screens ultraviolet radiation and includes at least one

optionally neutralized acid radical, and (ii) at least one insoluble filler which is inert and adsorbent with regard to the said agent.

2. Composition according to Claim 1, characterized in that the acid radical of the hydrophilic agent which screens ultraviolet radiation is a sulphonic radical - $SO_3H$.

3. Composition according to Claims 1 or 2, characterized in that the hydrophilic agent which screens ultraviolet radiation is a compound of structure 3-benzylidene camphor.

4. Composition according to Claim 3, characterized in that the hydrophilic agent which screens ultraviolet radiation corresponds to the following formula (I):

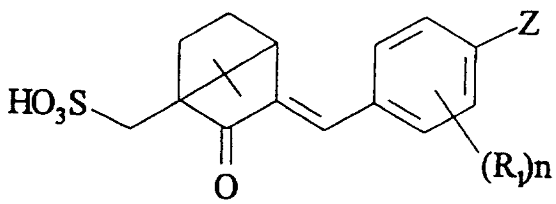

(I)

in which:

- Z denotes a group:

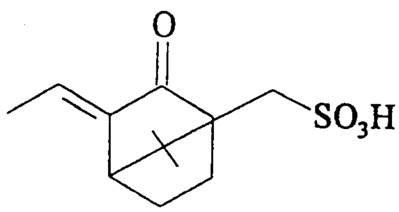

- n denotes 0 or an integer greater than or equal to 1 and less than or equal to 4,
- $R_1$ represents 1 or more identical or different, linear or branched alkyl or alkoxy radicals containing 1 to 4 carbon atoms.

5. Composition according to Claim 4, characterized in that the compound of formula (I) is benzene-1,4-di(3-methylidenecamphor-10-sulphonic) acid.

6. Composition according to Claim 3, characterized in that the hydrophilic agent which screens ultraviolet radiation corresponds to the following formula (II):

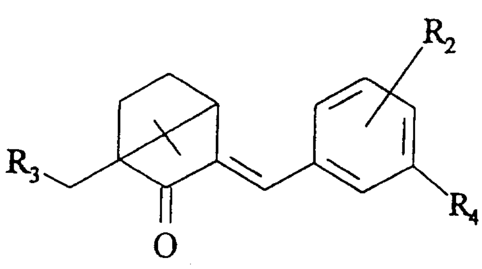

(II)

in which

- $R_2$ denotes a hydrogen atom, a halogen atom, an alkyl radical containing 1 to 4 carbon atoms or a $-SO_3H$ radical,
- $R_3$ and $R_4$ denote a hydrogen atom or a $-SO_3H$ radical, at least one of the radicals $R_2$, $R_3$ or $R_4$ denoting the $-SO_3H$ radical, it not being possible for $R_2$ and $R_4$ simultaneously to denote a $-SO_3H$ radical.

**7.** Composition according to Claim 6, characterized in that, in formula (II), $R_2$ denotes the $-SO_3H$ radical in a position para to the benzylidene camphor and $R_3$ and $R_4$ each represent a hydrogen atom.

**8.** Composition according to Claim 6, characterized in that, in formula (II), $R_2$ and $R_4$ each represent a hydrogen atom and $R_3$ denotes $-SO_3H$.

**9.** Composition according to Claim 6, characterized in that, in formula (II), $R_2$ denotes the methyl radical in a position para to the benzylidene camphor, $R_4$, radical, represents the $SO_3H$ radical and $R_3$ denotes a hydrogen atom.

**10.** Composition according to Claim 6, characterized in that, in formula (II), $R_2$ denotes a chlorine atom in a position para to the benzylidene camphor, $R_4$ denotes the radical $-SO_3H$ and $R_3$ denotes a hydrogen atom.

**11.** Composition according to Claim 6, characterized in that, in formula (II), $R_2$ denotes the methyl radical in a position para to the benzylidene camphor, $R_4$ denotes a hydrogen atom and $R_3$ denotes the $-SO_3H$ radical.

**12.** Composition according to Claim 3, characterized in that the hydrophilic agent which screens ultraviolet radiation corresponds to the following formula (III):

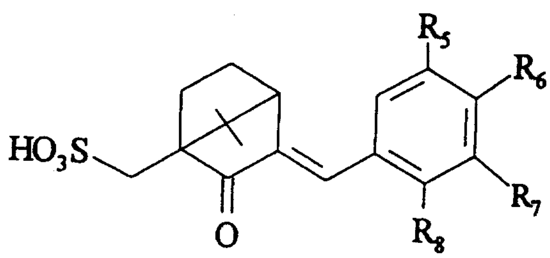

(III)

in which:

- $R_5$ and $R_7$, which are identical or different, denote a hydrogen atom, a hydroxyl radical, a linear or branched alkyl radical containing 1 to 8 carbon atoms or a linear or branched alkoxy radical containing 1 to 8 carbon atoms, at least one of the radicals $R_5$ and $R_7$ representing a hydroxyl, alkyl or alkoxy radical,
- $R_6$ and $R_8$, which are identical or different, denote a hydrogen atom or a hydroxyl radical, at least one of the radicals $R_6$ and $R_8$ denoting the hydroxyl radical,
- on condition that, when $R_5$ and $R_8$ denote hydrogen and $R_6$ denotes the hydroxyl radical, $R_7$ does not denote an alkoxy radical or a hydrogen atom.

**13.** Composition according to Claim 12, characterized in that, in formula (III), $R_5$ is a methyl radical, $R_6$ is a hydrogen atom, $R_7$ is a tert-butyl radical and $R_8$ is a hydroxyl radical.

**14.** Composition according to Claim 12, characterized in that, in formula (III), $R_5$ is a methoxy radical, $R_6$ is a hydrogen atom, $R_7$ is a tert-butyl radical and $R_8$ is a hydroxyl radical.

**15.** Composition according to Claim 12, characterized in that, in formula (III), $R_5$ and $R_7$ each denote a tert-butyl radical, $R_6$ denotes a hydroxyl radical and $R_8$ denotes a hydrogen atom.

**16.** Composition according to Claim 3, characterized in that the hydrophilic agent which screens ultraviolet radiation corresponds to the following formula (IV):

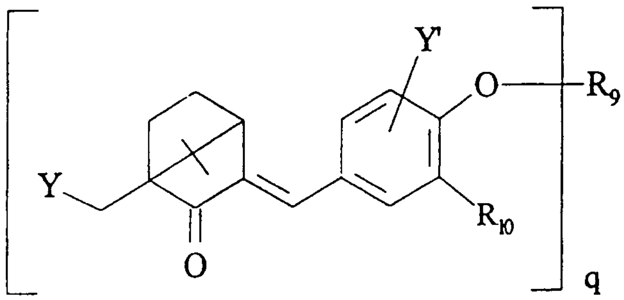

(IV)

in which

- $R_9$ denotes a hydrogen atom, a linear or branched alkyl radical containing 1 to 18 carbon atoms, a linear or branched alkenyl radical containing 3 to 18 carbon atoms, a group chosen from:

$$-CH_2-\underset{\displaystyle CH_2OH}{\underset{|}{CHOH}}\quad ,$$

or $-(CH_2CH_2O)_n-H$, or $-CH_2-CHOH-CH_3$, or $-(CH_2)_m-$ or $-CH_2-CHOH-CH_2-$, n being an integer between 1 and 6 ($1 \leq n \leq 6$) and m an integer between 1 and 10 ($1 \leq m \leq 10$),
- $R_{10}$ denotes a hydrogen atom, an alkoxy radical containing 1 to 4 carbon atoms, or a divalent -O- radical bonded to the $R_9$ radical when the latter is also divalent,
- q denotes an integer equal to 1 or 2, it being understood that if q is equal to 2, $R_9$ must denote a divalent radical.
- Y and Y' denote a hydrogen atom or a $-SO_3H$ radical, at least one of these radicals Y or Y' is different from hydrogen.

**17.** Composition according to Claim 16, characterized in that, in formula (IV), q is equal to 1, Y and $R_{10}$ each denote a hydrogen atom, $R_9$ denotes a methyl radical, Y' in position 3 denotes a $-SO_3H$ radical.

**18.** Composition according to Claim 16, characterized in that, in formula (IV), q is equal to 1, Y denotes a $-SO_3H$ radical, Y' denotes a hydrogen atom, $R_{10}$ denotes a divalent -O- radical bonded to $R_9$ which denotes a methylene radical.

**19.** Composition according to Claim 16, characterized in that, in formula (IV), q is equal to 1, Y denotes a - $SO_3H$ radical, Y' and $R_{10}$ both denote a hydrogen atom, and $R_9$ denotes a methyl radical.

**20.** Composition according to Claim 16, characterized in that, in formula (IV), q is equal to 1, Y denotes a - $SO_3H$ radical, Y' denotes a hydrogen atom, $R_9$ denotes a methyl radical and $R_{10}$ denotes a methoxy radical.

**21.** Composition according to Claim 16, characterized in that, in formula (IV), q is equal to 1, Y denotes a - $SO_3H$ radical, Y' and $R_{10}$ both denote a hydrogen atom, and $R_9$ denotes a n-butyl radical.

**22.** Composition according to Claim 16, characterized in that, in formula (IV), q is equal to 1, Y denotes a - $SO_3H$ radical, Y' denotes a hydrogen atom, $R_9$ denotes a n-butyl radical and $R_{10}$ denotes a methoxy radical.

**23.** Composition according to Claim 3, characterized in that the hydrophilic agent which screens ultraviolet radiation corresponds to the following formula (V):

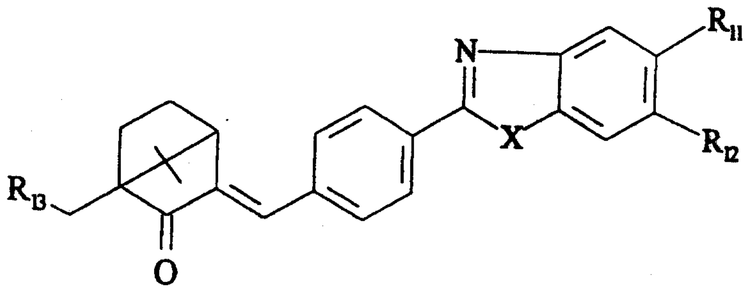

(V)

in which:

- $R_{11}$ denotes a hydrogen atom or a linear or branched alkyl or alkoxy radical containing 1 to 6 carbon atoms or a $-SO_3H$ radical,
- $R_{12}$ denotes a hydrogen atom or a linear or branched alkyl or alkoxy radical containing 1 to 6 carbon atoms,
- $R_{13}$ denotes a hydrogen atom or a $-SO_3H$ radical,
- at least one of the radicals $R_{11}$ or $R_{13}$ denotes a $-SO_3H$ radical,
- X is an oxygen or sulphur atom or a -NR- group, R being a hydrogen atom or a linear or branched alkyl radical containing 1 to 6 carbon atoms.

**24.** Composition according to Claim 23, characterized in that, in formula (V), X denotes a -NH- radical, $R_{11}$ denotes a $-SO_3H$ radical, and $R_{12}$ and $R_{13}$ both denote a hydrogen atom.

**25.** Composition according to Claims 1 or 2, characterized in that the hydrophilic agent which screens ultraviolet radiation is a compound of formula (VI):

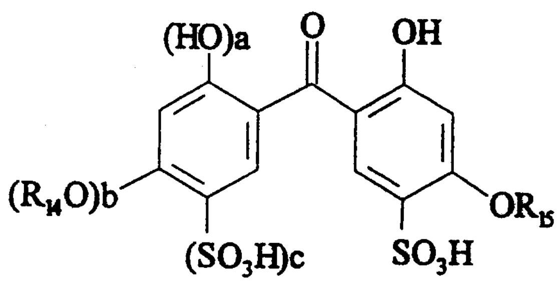

(VI)

in which

- $R_{14}$ and $R_{15}$, which are identical or different, denote a hydrogen atom or a linear or branched alkyl radical containing 1 to 8 carbon atoms,
- a, b and c, which are identical or different, are equal to 0 or 1.

**26.** Composition according to Claim 25, characterized in that, in formula (VI), a=b=c=0 and $R_{15}$ denotes a methyl radical.

**27.** Composition according to Claims 1 or 2, characterized in that the hydrophilic agent which screens ultraviolet radiation is a compound of formula (VII)

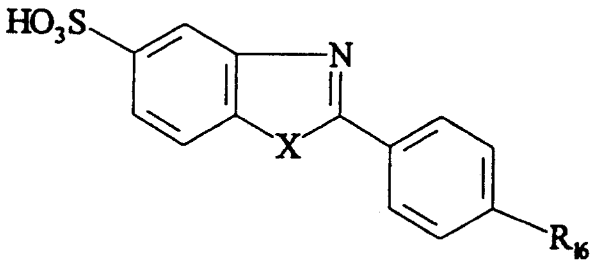

( VII )

in which:

- X denotes an oxygen atom or a -NH- radical,
- $R_{16}$ denotes a hydrogen atom, a linear or branched alkyl or alkoxy radical containing 1 to 8 carbon atoms or a group of formula (VIII)

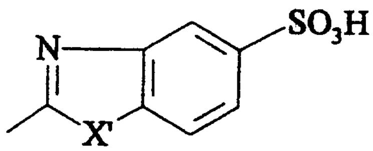

(VIII)

in which X' denotes, independently of X, an oxygen atom or a -NH- radical.

**28.** Composition according to Claim 27, characterized in that, in formula (VII), X denotes the -NH- radical and $R_{16}$ denotes a hydrogen atom.

**29.** Composition according to Claim 27, characterized in that, in formula (VII), X denotes the -NH- radical and $R_{16}$ denotes a group of formula (VIII) with X' denoting the -NH- radical.

**30.** Composition according to Claim 27 characterized in that, in formula (VII), X denotes an oxygen atom and $R_{16}$ denotes the group of formula (VIII) with X' denoting an oxygen atom.

**31.** Composition according to any one of the preceding claims, characterized in that the specific surface of the said inert adsorbent filler is at least 10 $m^2/g$, preferably at least 50 $m^2/g$ and even more preferably at least 100 $m^2/g$.

**32.** Composition according to any one of the preceding claims, characterized in that the said inert adsorbent filler is chosen from the group consisting of silicas, aluminas, aluminosilicates, zeolites, talc and magnesia.

**33.** Composition according to Claim 32, characterized in that the inert adsorbent filler is a fumed or precipitated adsorbent silica or a silica gel.

**34.** Composition according to any one of the preceding claims, characterized in that it contains approximately 0.2 to 10% by weight of hydrophilic agent which screens ultraviolet radiation, and preferably approximately 0.25 to 6% by weight with respect to the total weight of the composition.

**35.** Composition according to any one of the preceding claims, characterized in that it contains the inert adsorbent filler in a proportion of between approximately 0.15 and 5% by weight, and preferably between approximately 0.2 and 3% by weight, with respect to the total weight of the composition.

**36.** Composition according to any one of the preceding claims, characterized in that it furthermore contains one or more complementary hydrophilic or lipophilic sunscreens which are active in the UVB and/or UVA ranges, other than the acidic hydrophilic screens according to Claim 1.

37. Composition according to Claim 36, characterized in that the complementary sunscreens are chosen from cinnamates, salicylates, derivatives of benzylidene camphor, derivatives of triazine, derivatives of benzophenone, derivatives of dibenzoyl methane, derivatives of β,β-diphenylacrylate, derivatives of p-aminobenzoic acid, menthylanthranilate, screening polymers and screening silicones.

38. Composition according to Claim 37, characterized in that the complementary sunscreens are chosen from the group comprising 4-tert-butyl-4'-methoxydibenzoylmethane, 2-ethylhexyl-2-cyano-3,3-diphenylacrylate and 3-(4-methylbenzylidene)-camphor.

39. Composition according to any one of the preceding claims, characterized in that it furthermore contains, as complementary photoprotective agents, coated or uncoated metal oxide nanopigments capable of physically blocking UV radiation by scattering and/or reflection.

40. Composition according to any one of the preceding claims, characterized in that it furthermore comprises cosmetic adjuvants chosen from fats, organic solvents, nonionic thickeners, emollients, antioxidants, opacifying agents, stabilizers, silicones, anti-foaming agents, moisturisers, fragrances, preservatives, nonionic surfactants, fillers, sequestering agents, nonionic polymers, propellants, basifying or acidifying agents and dyes.

41. Composition according to any one of the preceding claims, characterized in that it constitutes a protective composition for the human epidermis or an antisun composition.

42. Composition according to any one of Claims 1 to 40, characterized in that it constitutes a make-up composition for the eyelashes, eyebrows or skin.

43. Composition according to any one of Claims 1 to 40, characterized in that it constitutes a composition for protecting the hair against ultraviolet rays.

44. Use of the compositions defined in any one of the preceding claims as, or for the manufacture of, cosmetic compositions for protecting the skin and/or the hair against ultraviolet radiation, in particular solar radiation.

45. Cosmetic treatment method for protecting the skin and/or the hair against ultraviolet radiation, in particular solar radiation, characterized in that it consists in applying thereto an effective quantity of a composition as defined in any one of Claims 1 to 43.

46. Use of an inert insoluble adsorbent filler, in particular a silica, in order to improve the water resistance of an antisun cosmetic composition of the oil-in-water emulsion type which contains, as photoprotective agent, a hydrophilic UV screen including at least one acid, in particular sulphonic, radical.

**Patentansprüche**

1. Filternde kosmetische Zusammensetzung, dadurch gekennzeichnet, daß sie in einem kosmetisch akzeptablen Träger vom Typ der Öl-in-Wasser-Emulsionen (i) mindestens ein hydraphiles Mittel, das die Ultraviolett-Strahlung zu filtern vermag und mindestens eine Säuregruppe aufweist, und (ii) mindestens einen inerten unlöslichen und diesem Mittel gegenüber adsorbierend wirkenden Füllstoff enthält.

2. Zusammensetzung nach Anspruch 1, dadurch gekennzeichnet, daß die Säuregruppe des hydrophilen Mittels, das die UV-Strahlung filtert, eine Sulfonsäuregruppe $-SO_3H$ ist.

3. Zusammensetzung nach Anspruch 1 oder 2, dadurch gekennzeichnet, daß das die UV-Strahlung filternde hydrophile Mittel eine Verbindung mit 3-Benzyliden-2-campher-Struktur ist.

4. Zusammensetzung nach Anspruch 3, dadurch gekennzeichnet, daß das die UV-Strahlung filternde hydrophile Mittel der folgenden Formel (I)

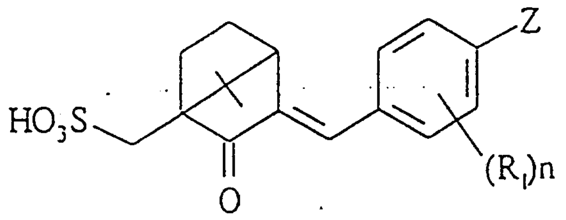

(I)

entspricht, in der bedeuten:

- Z eine Gruppe

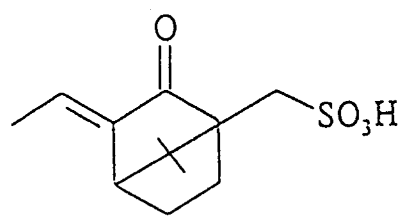

- n 0 oder eine ganze Zahl gleich oder größer als 1 und kleiner als oder gleich 4,
- $R_1$ eine oder mehrere geradkettige oder verzweigte, gleiche oder verschiedene Alkyl- oder Alkoxygruppen mit 1 bis 4 Kohlenstoffatomen.

5. Zusammensetzung nach Anspruch 4, dadurch gekennzeichnet, daß es sich bei der Verbindung der Formel (I) um Benzol-1,4-di-(3-methylidencampher-10-sulfonsäure) handelt.

6. Zusammensetzung nach Anspruch 3, dadurch gekennzeichnet, daß das die UV-Strahlung filternde hydrophile Mittel der folgenden Formel (II)

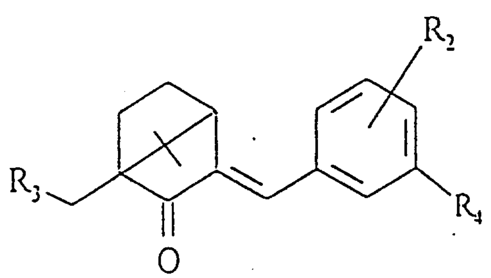

(II)

entspricht, in der bedeuten:

- $R_2$ ein Wasserstoffatom, ein Halogenatom, eine Alkylgruppe mit 1 bis 4 Kohlenstoffatomen, eine Gruppe -$SO_3H$,
- $R_3$ und $R_4$ ein Wasserstoffatom oder eine Gruppe -$SO_3H$, wobei mindestens eine der Gruppen $R_2$, $R_3$ und $R_4$ die Gruppe -$SO_3H$ ist und wobei $R_2$ und $R_4$ nicht gleichzeitig eine Gruppe -$SO_3H$ bedeuten können.

7. Zusammensetzung nach Anspruch 6, dadurch gekennzeichnet, daß in der Formel (II) $R_2$ die Gruppe -$SO_3H$ in p-Stellung des Benzylidencamphers bedeutet und $R_3$ und $R_4$ jeweils ein Wasserstoffatom darstellen.

8. Zusammensetzung nach Anspruch 6, dadurch gekennzeichnet, daß in der Formel (II) $R_2$ und $R_4$ jeweils ein Was-

serstoffatom bedeuten und $R_3$ -$SO_3H$ bedeutet.

**9.** Zusammensetzung nach Anspruch 6, dadurch gekennzeichnet, daß in der Formel (II) $R_2$ eine Methylgruppe in p-Stellung des Benzylidencamphers bedeutet, $R_4$ die Gruppe -$SO_3H$ und $R_3$ ein Wasserstoffatom darstellt.

**10.** Zusammensetzung nach Anspruch 6, dadurch gekennzeichnet, daß in der Formel (II) $R_2$ ein Chloratom in p-Stellung des Benzylidencamphers, $R_4$ die Gruppe -$SO_3H$ und $R_3$ ein Wasserstoffatom bedeutet.

**11.** Zusammensetzung nach Anspruch 6, dadurch gekennzeichnet, daß in der Formel (II) $R_2$ eine Methylgruppe in p-Stellung des Benzylidencamphers bedeutet, $R_4$ ein Wasserstoffatom und $R_3$ die Gruppe -$SO_3H$ bedeutet.

**12.** Zusammensetzung nach Anspruch 3, dadurch gekennzeichnet, daß das die UV-Strahlung filternde hydrophile Mittel der folgenden Formel (III)

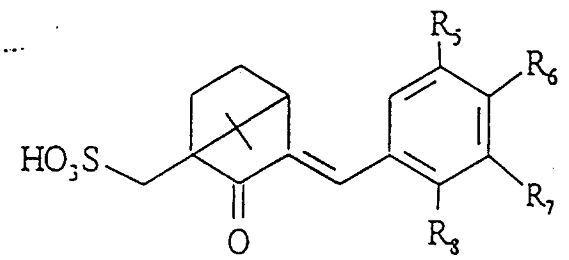

(III)

entspricht, in der bedeuten:

- $R_5$ und $R_6$, die gleich oder verschieden sind, ein Wasserstoffatom, eine Hydroxygruppe, eine geradkettige oder verzweigte Alkylgruppe mit 1 bis 8 Kohlenstoffatomen oder eine geradkettige oder verzweigte Alkoxygruppe mit 1 bis 8 Kohlenstoffatomen, wobei mindestens eine der Gruppen $R_5$ und $R_7$ eine Hydroxy-, Alkyl- oder Alkoxygruppe darstellt,
- $R_6$ und $R_8$, die gleich oder verschieden sind, ein Wasserstoffatom, eine Hydroxygruppe, wobei mindestens eine der Gruppen $R_6$ und $R_8$ die Hydroxygruppe darstellt,
- mit der Maßgabe, daß, wenn $R_5$ und $R_8$ Wasserstoff bedeuten und $R_6$ die Hydroxygruppe bedeutet, $R_7$ weder eine Alkoxygruppe noch ein Wasserstoffatom ist.

**13.** Zusammensetzung nach Anspruch 12, dadurch gekennzeichnet, daß in der Formel (III) $R_5$ eine Methylgruppe, $R_6$ ein Wasserstoffatom, $R_7$ eine *tert.*-Butylgruppe und $R_8$ eine Hydroxygruppe ist.

**14.** Zusammensetzung nach Anspruch 12, dadurch gekennzeichnet, daß in der Formel (III) $R_5$ eine Methoxygruppe, $R_6$ ein Wasserstoffatom, $R_7$ eine *tert.*-Butylgruppe und $R_8$ eine Hydroxygruppe ist.

**15.** Zusammensetzung nach Anspruch 12, dadurch gekennzeichnet, daß in der Formel (III) $R_5$ und $R_7$ jeweils eine *tert.*-Butylgruppe bedeuten, $R_6$ eine Hydroxygruppe und $R_8$ ein Wasserstoffatom ist.

**16.** Zusammensetzung nach Anspruch 3, dadurch gekennzeichnet, daß das die UV-Strahlung filternde hydrophile Mittel der folgenden Formel (IV)

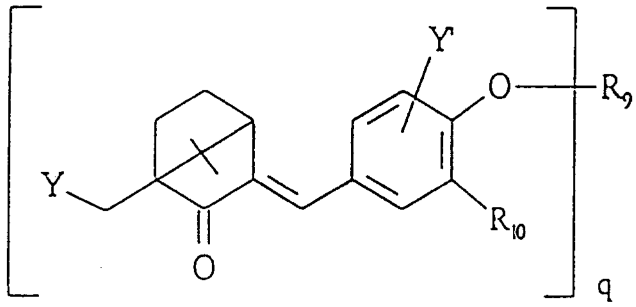

(IV)

entspricht, in der bedeuten:

- $R_9$ ein Wasserstoffatom, eine geradkettige oder verzweigte Alkylgruppe mit 1 bis 18 Kohlenstoffatomen, eine geradkettige oder verzweigte Alkenylgruppe mit 3 bis 18 Kohlenstoffatomen, eine Gruppe, die ausgewählt ist unter

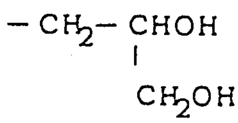
,

$-(CH_2CH_2O)_n-H$ , $-CH_2-CHOH-CH_3$ , $-(CH_2)_m-$ und $-CH_2-CHOH-CH_2-$, wobei n eine ganze Zahl von 1 bis 6 ($1 \leq n \leq 6$) und m eine ganze Zahl von 1 bis 10 ($1 \leq m \leq 10$) ist,
- $R_{10}$ ein Wasserstoffatom, eine Alkoxygruppe mit 1 bis 4 Kohlenstoffatomen oder ein zweiwertiger Rest -O-, der mit der Gruppe $R_9$ verbunden ist, wenn diese ebenfalls zweiwertig ist,
- q die ganze Zahl 1 oder 2, wobei $R_9$ eine zweiwertige Gruppe sein muß, wenn q gleich 2 ist,
- Y und Y' ein Wasserstoffatom oder eine Gruppe $-SO_3H$, wobei mindestens eine dieser Gruppen Y und Y' von Wasserstoff verschieden ist.

**17.** Zusammensetzung nach Anspruch 16, dadurch gekennzeichnet, daß in der Formel (IV) q gleich 1 ist, Y und $R_{10}$ jeweils ein Wasserstoffatom bedeuten, $R_9$ eine Methylgruppe und Y' in 3-Stellung eine Gruppe $-SO_3H$ bedeutet.

**18.** Zusammensetzung nach Anspruch 16, dadurch gekennzeichnet, daß in der Formel (IV) q gleich 1 ist, Y eine Gruppe $-SO_3H$, Y' ein Wasserstoffatom, $R_{10}$ einen zweiwertigen Rest -O- bedeutet, der mit $R_9$ verbunden ist, das eine Methylengruppe bedeutet.

**19.** Zusammensetzung nach Anspruch 16, dadurch gekennzeichnet, daß in der Formel (IV) q gleich 1 ist, Y eine Gruppe $-SO_3H$ bedeutet, Y' und $R_{10}$ jeweils ein Wasserstoffatom bedeuten und $R_9$ eine Methylgruppe ist.

**20.** Zusammensetzung nach Anspruch 16, dadurch gekennzeichnet, daß in der Formel (IV) q gleich 1 ist, Y eine Gruppe $-SO_3H$, Y' ein Wasserstoffatom, $R_9$ eine Methylgruppe und $R_{10}$ eine Methoxygruppe bedeutet.

**21.** Zusammensetzung nach Anspruch 16, dadurch gekennzeichnet, daß in der Formel (IV) q gleich 1 ist, Y eine Gruppe $-SO_3H$ bedeutet, Y' und $R_{10}$ jeweils ein Wasserstoffatom bedeuten und $R_9$ eine n-Butylgruppe ist.

**22.** Zusammensetzung nach Anspruch 16, dadurch gekennzeichnet, daß in der Formel (IV) q gleich 1 ist, Y eine Grupe $-SO_3H$, Y' ein Wasserstoffatom, $R_9$ eine n-Butylgruppe und $R_{10}$ eine Methoxygruppe bedeutet.

**23.** Zusammensetzung nach Anspruch 3, dadurch gekennzeichnet, daß das die UV-Strahlung filternde hydrophile Mittel der folgenden Formel (V)

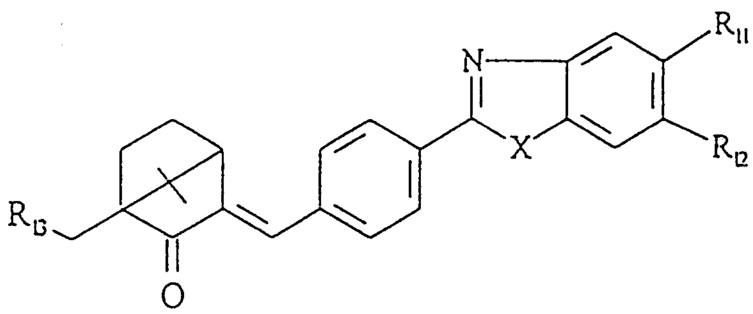

(V)

entspricht, in der bedeuten:

- $R_{11}$ ein Wasserstoffatom, eine geradkettige oder verzweigte Alkyl- oder Alkoxygruppe mit 1 bis 6 Kohlenstoffatomen, eine Gruppe $-SO_3H$,
- $R_{12}$ ein Wasserstoffatom, eine geradkettige oder verzweigte Alkyl- oder Alkoxygruppe mit 1 bis 6 Kohlenstoffatomen,
- $R_{13}$ ein Wasserstoffatom oder eine Gruppe $-SO_3H$,
- mindestens eine der Gruppen $R_{11}$ und $R_{13}$ eine Gruppe $-SO_3H$,
- X ein Sauerstoff- oder Schwefelatom oder eine Gruppe -NR-, wobei R ein Wasserstoffatom oder eine geradkettige oder verzweigte Alkylgruppe mit 1 bis 6 Kohlenstoffatomen ist.

**24.** Zusammensetzung nach Anspruch 23, dadurch gekennzeichnet, daß in der Formel (V) X eine Gruppe -NH- bedeutet, $R_{11}$ eine Gruppe $-SO_3H$ ist und $R_{12}$ und $R_{13}$ jeweils ein Wasserstoffatom bedeuten.

**25.** Zusammensetzung nach Anspruch 1 der 2, dadurch gekennzeichnet, daß das die UV-Strahlung filternde Mittel eine Verbindung der Formel (VI)

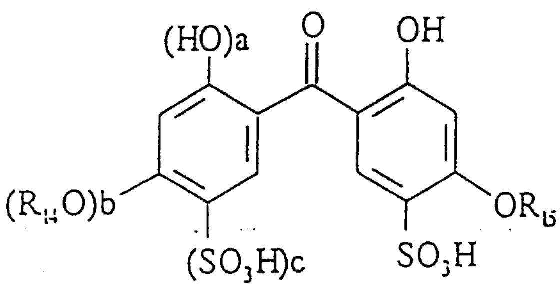

(VI)

ist, in der bedeuten:

- $R_{14}$ und $R_{15}$, die gleich oder verschieden sind, ein Wasserstoffatom oder eine geradkettige oder verzweigte Alkylgruppe mit 1 bis 8 Kohlenstoffatomen,
- a, b und c die gleich oder verschieden sind, 0 oder 1.

**26.** Zusammensetzung nach Anspruch 25, dadurch gekennzeichnet, daß in der Formel (VI) a=b=c=0 ist und $R_{15}$ eine Methylgruppe bedeutet.

**27.** Zusammensetzung nach Anspruch 1 oder 2, dadurch gekennzeichnet, daß das die UV-Strahlung filternde hydrophile Mittel eine Verbindung der Formel (VII)

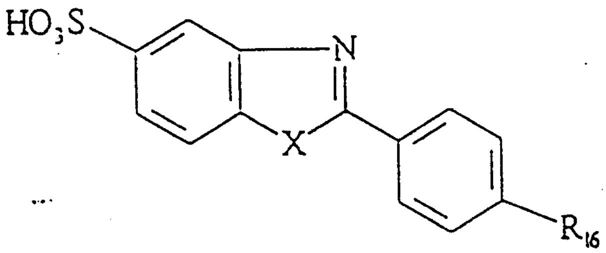

(VII)

ist, in der bedeuten:

- X ein Sauerstoffatom oder eine Gruppe -NH-,
- $R_{16}$ ein Wasserstoffatom, eine geradkettige oder verzweigte Alkyl- oder Alkoxygruppe mit 1 bis 8 Kohlenstoffatomen oder eine Gruppe der Formel (VIII)

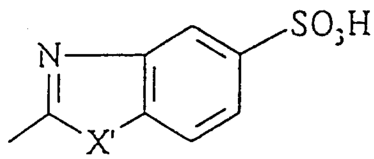

(VIII),

in der X' unabhängig von X ein Sauerstoffatom oder eine Gruppe -NH- bedeutet.

**28.** Zusammensetzung nach Anspruch 27, dadurch gekennzeichnet, daß in der Formel (VII) X die Gruppe -NH- und $R_{16}$ ein Wasserstoffatom bedeutet.

**29.** Zusammensetzung nach Anspruch 27, dadurch gekennzeichnet, daß in der Formel (VII) X die Gruppe -NH- und $R_{16}$ die Gruppe der Formel (VIII) bedeutet, in der X' die Gruppe -NH- ist.

**30.** Zusammensetzung nach Anspruch 27, dadurch gekennzeichnet, daß in der Formel (VII) X ein Sauerstoffatom ist, $R_{16}$ die Gruppe der Formel (VIII) bedeutet, in der X' ein Sauerstoffatom ist.

**31.** Zusammensetzung nach einem der vorhergehenden Ansprüche, dadurch gekennzeichnet, daß die spezifische Oberfläche des adsorbierenden inerten Füllstoffs mindestens 10 $m^2/g$, vorzugsweise mindestens 50 $m^2/g$ und noch bevorzugter mindestens 100 $m^2/g$ beträgt.

**32.** Zusammensetzung nach einem der vorhergehenden Ansprüche, dadurch gekennzeichnet, daß der adsorbierende inerte Füllstoff unter Kieselsäuren, Aluminiumoxiden, Aluminosilicaten, Zeolithen, Talk und Magnesiumoxid ausgewählt ist.

**33.** Zusammensetzung nach Anspruch 32, dadurch gekennzeichnet, daß der adsorbierende inerte Füllstoff eine adsorbierende pyrogene Kieselsäure, eine adsorbierende Fällungskieselsäure oder ein Kieselsäuregel ist.

**34.** Zusammensetzung nach einem der vorhergehenden Ansprüche, dadurch gekennzeichnet, daß sie etwa 0,2 bis 10 Gew.-% die UV-Strahlung filterndes hydrophiles Mittel und vorzugsweise etwa 0,25 bis 6 Gew.-%, bezogen auf das Gesamtgewicht der Zusammensetzung, enthält.

**35.** Zusammensetzung nach einem der vorhergehenden Ansprüche, dadurch gekennzeichnet, daß sie den adsorbierenden inerten Füllstoff in einem Anteil von etwa 0,15 bis 5 Gew.-% und vorzugsweise etwa 0,2 bis 3 Gew.-%, bezogen auf das Gesamtgewicht der Zusammensetzung, enthält.

**36.** Zusammensetzung nach einem der vorhergehenden Ansprüche, dadurch gekennzeichnet, daß sie außerdem einen oder mehrere ergänzende, im UV-B und/oder UV-A wirksame, hydrophile oder lipophile Lichtschutzfilter enthält, die von den sauren hydrophilen Filtern gemäß Anspruch 1 verschieden sind.

**37.** Zusammensetzung nach Anspruch 36, dadurch gekennzeichnet, daß die ergänzenden Lichtschutzfilter ausgewählt sind unter Cinnamaten, Salicylaten, Benzylidencampher-Derivaten, Triazin-Derivaten, Benzophenon-Derivaten, Dibenzoylmethan-Derivaten, β,β-Diphenylacrylat-Derivaten, p-Aminobenzoesäure-Derivaten, Menthylanthranilat, Polymerfiltern und Siliconfiltern.

**38.** Zusammensetzung nach Anspruch 37, dadurch gekennzeichnet, daß die ergänzenden Lichtschutzfilter unter 4-*tert*.-Butyl-4'-methoxydibenzoylmethan, 2-Ethylhexyl-2-cyano-3,3-diphenylacrylat und 3-(4-Methylbenzyliden)campher ausgewählt sind.

**39.** Zusammensetzung nach einem der vorhergehenden Ansprüche, dadurch gekennzeichnet, daß sie als ergänzende Lichtschutzmittel gegebenenfalls umhüllte Nanopigmente aus Metalloxiden enthält, die die UV-Strahlung physikalisch durch Diffusion und/oder Reflexion abzublocken vermögen.

**40.** Zusammensetzung nach einem der vorhergehenden Ansprüche, dadurch gekennzeichnet, daß sie außerdem kosmetische Hilfsstoffe enthält, die unter Fettsubstanzen, organischen Lösungsmitteln, nichtionischen Verdickungsmitteln, reizlindernden Mitteln, Antioxidantien, Trübungsmitteln, Stabilisierungsmitteln, Siliconen, Antischaummitteln, Hydratisierungsmitteln, Parfums, Konservierungsmitteln, nichtionischen grenzflächenaktiven Stoffen, Füllstoffen, Maskierungsmitteln, nichtionischen Polymeren, Treibmitteln, Alkalisierungsmitteln oder Säuerungsmitteln, Färbemitteln oder Farbstoffen ausgewählt sind.

**41.** Zusammensetzung nach einem der vorhergehenden Ansprüche, dadurch gekennzeichnet, daß sie eine die Epidermis des Menschen schützende Zusammensetzung oder eine Lichtschutzzusammensetzung ist.

**42.** Zusammensetzung nach einem der Ansprüche 1 bis 40, dadurch gekennzeichnet, daß sie eine Schminkzusammensetzung für die Wimpern, die Augenbrauen oder die Haut ist.

**43.** Zusammensetzung nach einem der Ansprüche 1 bis 40, dadurch gekennzeichnet, daß sie eine Zusammensetzung für den Schutz der Haare vor UV-Strahlung ist.

**44.** Verwendung der in einem der vorhergehenden Ansprüche definierten Zusammensetzungen als kosmetische Zusammensetzungen oder für die Herstellung kosmetischer Zusammensetzungen für den Schutz der Haut und/oder der Haare vor UV-Strahlung und insbesondere vor Sonnenlicht.

**45.** Verfahren zur kosmetischen Behandlung zum Schutz der Haut und/oder der Haare vor UV-Strahlung, insbesondere vor Sonnenlicht, dadurch gekennzeichnet, daß es darin besteht, auf die Haut und/oder die Haare eine wirksame Menge einer wie in einem der Ansprüche 1 bis 43 definierten Zusammensetzung aufzutragen.

**46.** Verwendung eines adsorbierenden, unlöslichen, inerten Füllstoffs, insbesondere einer Kieselsäure, zur Verbesserung der Wasserfestigkeit einer kosmetischen Lichtschutzzusammensetzung vom Typ der Öl-in-Wasser-Emulsionen, die als Lichtschutzmittel ein hydrophiles UV-Filter enthält, das mindestens eine Säuregruppe, insbesondere eine Sulfonsäuregruppe, enthält.